# EUROPÄISCHE PATENTSCHRIFT

(11) **EP 3 966 529 B1**
(45) Veröffentlichungstag und Bekanntmachung des Hinweises auf die Patenterteilung: **18.11.2026**
(21) Anmeldenummer: 20725671.0
(22) Anmeldetag: 06.05.2020
(51) Int. Cl.: G01D 21/00, G01D 11/30, A61B 5/00, A61B 5/11

(54) **SENSORMODUL, PFLEGESET UND VERWENDUNG HIERFÜR**
SENSOR MODULE, CARE SET AND USE THEREOF
MODULE DE CAPTEUR, ENSEMBLE DE SOINS ET LEUR UTILISATION

(30) Priorität: 09.05.2019 DE 102019112126
(43) Veröffentlichungstag der Anmeldung: 16.03.2022
(73) Patentinhaber: Ahead Care GmbH, 91052 Erlangen (DE)
(72) Erfinder: BESSER, Jürgen, 90587 Tuchenbach (DE); HEFNER, Günther Maximilian, 85521 Riemerling (DE)
(74) Vertreter: Geling, Andrea
(86) Internationale Anmeldenummer: PCT/EP2020/062569
(87) Internationale Veröffentlichungsnummer: WO 2020/225298

(56) Entgegenhaltungen:
- DE-A1- 102005 004 086
- DE-A1- 102017 107 864
- DE-U1- 202016 103 635
- US-A1- 2015 351 690
- US-A1- 2017 215 766

## Beschreibung

### HINTERGRUND DER ERFINDUNG

### Gebiet der Erfindung

Die Erfindung betrifft ein Sensormodul, ein Pflegeset sowie die Verwendung eines Teilmoduls zur Erfassung und Auswertung von gesundheitsrelevanten Daten von pflegebedürftigen Menschen.

### Stand der Technik

In der DE 10 2017 107 864 A1 ist eine Hautauflage offenbart, welche mit der Haut eines Trägers lösbar verbunden werden soll. Hierzu ist auf der der Haut zugewandten Seite eine Klebeschicht vorgesehen. Auf der hautabgewandten Seite ist ein Taschenabschnitt vorgesehen, in welchem ein Assistenzsystem mit elektronischen Komponenten aufgenommen werden kann.

DE 10 2005 004 086 A1 betrifft ein Gerät zur Ermittlung von Bewegungen. Das Gerät erfasst mittels mehreren Bewegungssensoren die Bewegungen im Raum. Die Messwerte mindestens einer Bewegungsdimension werden mit den Messwerten mindestens einer anderen Bewegungsdimension verrechnet. Eine Drehgeschwindigkeitsmessung wird mit einer linearen Beschleunigungsmessung kombiniert. Drei lineare Beschleunigungsmessungen werden kombiniert, um Beschleunigungen in drei korrigierte Raumrichtungen umzurechnen. Eine solche Transformation ist nützlich, wenn das Anbringen der Sensoren nach den zu messenden Raumrichtungen schwierig ist, insbesondere bei Messungen am menschlichen Körper. Die Beschleunigungssensoren können nach diesem Verfahren beliebig angebracht werden, gewünschte Richtungen werden mittels zwei Initialpositionen festgelegt.

DE 10 2017 107 864 A1 betrifft eine Hautauflage mit einem flächigen Trägermaterial, mit einer im Gebrauch der Haut eines Trägers der Hautauflage zugewandten Seite und einer der Haut abgewandten Seite, wobei das Trägermaterial auf der der Haut zugewandten Seite zumindest bereichsweise mit einer Klebeschicht versehen ist zum Fixieren der Hautauflage auf der Haut des Trägers, dadurch gekennzeichnet, dass die Hautauflage wenigstens einen ersten Taschenabschnitt aufweist, welcher von der der Haut abgewandten Seite zugänglich ist.

### ZUSAMMENFASSUNG DER ERFINDUNG

Aufgabe der vorliegenden Erfindung ist es, eine im Vergleich zum Stand der Technik verbesserte Vorrichtung anzugeben.

Diese Aufgabe wird durch ein Sensormodul mit den Merkmalen des Anspruchs 1, ein Pflegeset mit den Merkmalen des Anspruchs 12 und der Verwendung eines zweiten Teilmoduls mit den Merkmalen des Anspruchs 13 gelöst.

Erfindungsgemäß wird in einem ersten Aspekt ein Sensormodul vorgeschlagen, umfassend ein erstes Teilmodul mit einer ersten Struktur und mit einer elektronischen Einheit zur Erfassung und Übermittlung von Bewegungsdaten. Das erfindungsgemäße Sensormodul umfasst weiterhin ein zweites Teilmodul mit einem Befestigungsmittel zur Befestigung des zweiten Teilmoduls an einem Träger, und eine zweite Struktur, wobei die erste Struktur mit der zweiten Struktur eine reversible mechanische Verbindung ausbilden kann, so dass das erste Teilmodul und das zweite Teilmodul reversibel miteinander verbunden werden können.

Dadurch wird vorteilhaft erreicht, dass das erste Teilmodul, welches die elektronische Einheit enthält, von dem Träger entfernt werden kann, ohne das zweite Teilmodul von dem Träger entfernen zu müssen, welches durch das Befestigungsmittel an dem Träger befestigt ist. Hierdurch wird eine zusätzliche Flexibilität erreicht. Für einen etwaigen Austausch oder eine Reparatur des ersten Teilmoduls oder Teilen davon, wie beispielsweise der elektronischen Einheit, ist es nicht erforderlich, das gesamte Sensormodul von dem Träger abzunehmen. Dies ist auch für den Träger, an welchem das zweite Teilmodul befestigt ist, angenehmer, da die Befestigung nicht entfernt und erneut angebracht werden muss. Vielmehr ist es ausreichend, die reversible Verbindung zwischen der ersten Struktur des ersten Teilmoduls und der zweiten Struktur des zweiten Teilmoduls zu lösen, um das erste und zweite Teilmodul voneinander zu trennen.

Die erste Struktur umfasst eines aus der Gruppe Haken-Struktur oder Schlaufen-Struktur, und die zweite Struktur umfasst das andere aus der Gruppe Haken-Struktur oder Schlaufen-Struktur, welche beim Zusammendrücken der ersten Struktur mit der zweiten Struktur eine reversible mechanische Verbindung ausbilden. Die reversible mechanische Verbindung ist als reversibler Formschluss, insbesondere als Klettverschluss ausgebildet. Dadurch wird eine zuverlässige lösbare Befestigung sichergestellt, welche das erste Teilmodul mit dem zweiten Teilmodul lösbar verbindet.

Das Befestigungsmittel umfasst eine klebende Schicht, insbesondere eine klebende Schicht, welche an der menschlichen Haut haftbar ist. Dies hat den Vorteil, dass das Sensormodul direkt an einem Hautbereich eines Trägers stoffschlüssig angebracht werden kann. Dadurch, dass das erste Teilmodul und das zweite Teilmodul durch eine reversible mechanische Verbindung verbunden sind, ist es für die Abnahme des ersten Teilmoduls nicht erforderlich, die Klebeschicht, durch welche das zweite Teilmodul am Körper des Trägers befestigt ist, zu lösen. Ein Vermeiden des Lösens und Wiederanbringens der Klebeschicht führt auch zu einer längeren Haftwirkung der Klebeschicht, und die Erneuerung der klebenden Schicht bzw. des zweiten Teilmoduls können jeweils nach einem längeren Zeitintervall erfolgen. Dies ist auch vorteilhaft für den Träger, der das Entfernen der Klebeschicht weniger oft über sich ergehen lassen muss.

Zweckmäßigerweise umfasst das zweite Teilmodul einen dehnbaren Klebestreifen. Dadurch kann sich das zweite Teilmodul optimal an die Geometrie der Hautoberfläche, auf welche das zweite Teilmodul aufgeklebt wird, anpassen. Dies ist besonders vorteilhaft, wenn der Träger in Bewegung ist und sich die Geometrie der Hautoberfläche dynamisch ändert.

Vorzugsweise umfasst das zweite Teilmodul eine Bauchbinde. Dies ermöglicht die Befestigung des zweiten Teilmoduls an dem Träger, welche mechanisch lösbar ist und keine Klebeschicht erfordert.

Bevorzugt umfasst die elektronische Einheit wenigstens einen Beschleunigungssensor zur Messung von Beschleunigungsdaten. Die gemessenen Beschleunigungsdaten werden zur Bewegungsauswertung des Trägers herangezogen. Dies kann beispielsweise die Erkennung eines Sturzes umfassen. Weiterhin können die Daten hinsichtlich Trageerkennung, Lageerkennung, sowie Dekubituserkennung ausgewertet werden.

Alternativ oder kumulativ umfasst die elektronische Einheit ein GPS-Modul zur Erfassung und Übermittlung von Ortskoordinaten des ersten Teilmoduls bzw. des Sensormoduls, wobei GPS für globales Positionssystem steht. Dadurch ist eine Ortung und eine Aktivitätsanalyse des Trägers möglich. Dies ist vorteilhaft, wenn beispielsweise die Auswertung der Beschleunigungsdaten einen Sturz des Trägers ergeben hat und der Ort des Trägers möglichst schnell ermittelt werden soll.

In einer weiteren vorteilhaften Ausgestaltung umfasst das zweite Teilmodul einen Nahfeldkommunikations-Sender zum Senden von Daten, und das erste Teilmodul einen Nahfeldkommunikations-Empfänger zum Empfangen der Daten, wobei die Daten zur Verifizierung
a) der Funktionalität der Paarung des ersten Teilmoduls mit dem zweiten Teilmodul, und/oder
b) der korrekten Anbringung des ersten Teilmoduls und/oder
c) der Verwendung eines proprietären zweiten Teilmoduls
herangezogen werden.

Der Datenaustausch in der Nahfeldkommunikation, beispielsweise über RFID, das Radiofrequenz-Identifikation bedeutet, erfolgt über elektromagnetische Induktion und ist daher nur bei geringen Reichweiten von wenigen Zentimetern bis etwa 10 cm möglich. Diese geringe Reichweite wird vorliegend zur Verifizierung der Paarung genutzt, dahingehend, ob das zweite Teilmodul in örtlicher Nähe zum ersten Teilmodul angeordnet bzw. mit diesem verbunden ist. Die Verifizierung der korrekten Anbringung stellt sicher, dass das Sensormodul hinsichtlich Vorder- und Rückseite / Ober- Unterseite richtig angeordnet ist, also das erste Teilmodul richtig ausgerichtet zum zweiten Teilmodul an diesem befestigt ist. Ferner wird durch die Übermittlung eines ID-Signales sichergestellt, dass als zweites Teilmodul ein verifiziertes Original-Teilmodul verwendet wird. Durch diese verifizierenden Schritte wird ein zuverlässiger Betrieb des Sensormoduls sowie die Richtigkeit der zu erfassenden und übertragenden Daten sichergestellt. Darüber hinaus sind die genannten Verifizierungsschritte notwendige Voraussetzungen, um die Funktionen des ersten Teilmoduls in Betrieb zu nehmen.

Vorzugsweise umfasst das erste Teilmodul einen kapazitiven Sensor zur Messung von Kapazitätswerten zwischen einer dem kapazitiven Sensor zugehörigen Kondensator-Elektrode und der Haut des Trägers, welche als zweite Elektrode wirkt. Der kapazitive Sensor ist bevorzugt auf der elektronischen Einheit angeordnet. Der kapazitive Sensor kann aber auch an einer anderen Position des ersten Teilmoduls angeordnet sein. In jedem Fall ist der kapazitive Sensor mit der elektronischen Einheit elektrisch verbunden, so dass die durch den kapazitiven Sensor gemessenen Kapazitätswerte an die elektronische Einheit übermittelt werden können. Bekanntermaßen ist die Kapazität eines Kondensators abhängig vom Abstand seiner Elektroden. Durch die Messung von Kapazitätswerten mit der vorliegenden Anordnung kann entsprechend bestimmt werden, ob das erste Teilmodul vom zweiten Teilmodul, das am Träger befestigt ist, getrennt wurde. Ebenfalls ist es möglich zu bestimmen, ob das Sensormodul vom Träger entfernt wurde. Denn auch in diesem Fall ändert sich die Kapazität.

Es ist vorteilhaft, wenn die elektronische Einheit eine Vergleichseinheit umfasst, welche ausgebildet ist, einen Kapazitäts-Referenzwert mit einem aktuellen Kapazitätswert zu vergleichen, und auszulösen, dass Daten nur dann gemessen, erfasst und/oder übermittelt werden wenn der aktuelle Kapazitätswert größer oder gleich dem Referenzwert ist. Bevorzugt handelt es sich bei den Daten um die Daten der Beschleunigung, der Ortskoordinaten sowie der Verifizierung. Der Referenzwert kann beispielsweise so gewählt werden, dass dieser mit dem Abstand des ersten Teilmoduls bzw. der Kondensator-Elektrode zur Hautoberfläche des Trägers korreliert wenn das Sensormodul an der Hautoberfläche des Trägers befestigt ist. Durch den Vergleich mit diesem Referenzwert kann zuverlässig bestimmt werden, ob das erste Teilmodul vom zweiten Teilmodul, das am Träger befestigt ist, getrennt wurde, oder ob das Sensormodul vom Träger entfernt wurde.

Es ist auch möglich, dass die Daten nur dann gemessen, erfasst und/oder übermittelt werden wenn der aktuelle Kapazitätswert kleiner oder gleich dem Referenzwert ist.

In einer weiteren vorteilhaften Ausgestaltung umfasst die elektronische Einheit ein Kommunikationsmodul, mit welchem die erfassten Daten an eine Auswerteeinheit außerhalb des Sensormoduls gesendet werden. Dies ermöglicht die gezielte Auswertung und Sichtung der Daten an einer zugeordneten Auswerteeinheit von einer entsprechenden Fachkraft. Das Kommunikationsmodul kann ein GSM-Kommunikationsmodul sein, wobei GSM globaler Mobilfunkstandard bedeutet.

Zweckmäßigerweise umfasst das erste Teilmodul eine flexible Leiterverbindung, welche die elektronische Einheit mit einer Leistungsquelle verbindet, welche die elektronische Einheit mit Energie versorgt. Dies hat den Vorteil, dass sich die Leiterverbindung den Bewegungen des Trägers anpassen kann, vorausgesetzt, das Sensormodul ist entsprechend an dem Träger angebracht. Das Sensormodul wird vorteilhaft derart am Rücken des Trägers angebracht, so dass die Leiterverbindung parallel oder senkrecht zur Wirbelsäule verläuft, um sich üblichen Bewegungen wie nach vorne beugen anzupassen.

Bevorzugt umfasst das erste Teilmodul eine Ummantelung aus einem flexiblen Material, welches vorzugsweise mittels eines Gießverfahrens die Leistungsquelle und die elektronische Einheit umschließt, so dass die Leistungsquelle und die elektronische Einheit mit der Ummantelung einen nichtlösbaren Formschluss ausbilden. Dadurch ist die Leistungsquelle und die elektronische Einheit vor mechanischen Einflüssen geschützt und zudem wird ein Loslösen der Leistungsquelle und/oder der elektronischen Einheit von der Ummantelung verhindert.

Zweckmäßigerweise umfasst die Ummantelung wenigstens einen flexiblen, vorzugsweise linearen, Bereich, um welche sich das erste Teilmodul biegen läßt, welcher eine Biegeachse ausbildet, um welchen sich das erste Teilmodul biegen läßt, so dass bei einer Biegung des ersten Teilmoduls um den linearen Bereich die flexible Leiterverbindung ebenfalls gebogen wird. Dies hat den Vorteil, dass sich das erste Teilmodul in der Nutzung den Bewegungen des Trägers anpassen kann, welcher das Sensormodul trägt.

In einem zweiten Aspekt der Erfindung wird ein Pflegeset vorgeschlagen, umfassend ein erfindungsgemäßes Sensormodul, sowie eine von dem Sensormodul getrennt angeordnete Auswerteeinheit mit einer Software, mit welcher die gemessenen Daten empfangen, ausgewertet und angezeigt werden. Alternativ kann das Pflegeset auch ein erfindungsgemäßes Sensormodul und ein Computerprogrammprodukt umfassen, das, wenn auf einer elektronischen Auswerteeinheit installiert und ausgeführt, eine Software bildet, mit welcher die gemessenen Daten empfangen, ausgewertet und angezeigt werden.

Das Empfangen und Auswerten erfolgt mittels einer separaten Auswerteeinheit mit einer Software durch eine Fachkraft, welche gegebenenfalls geeignete Maßnahmen aufgrund der ausgewerteten Daten einleiten kann. Beispielsweise ist es bei der Erkennung, dass das erste Teilmodul nicht mehr an dem zweiten Teilmodul befestigt ist, weil der Nahfeldkommunikations-Empfänger keine Daten mehr empfängt, erforderlich, das erste Teilmodul wieder am zweiten Teilmodul zu befestigen.

Bevorzugt wird das Computerprogrammprodukt in einem Computer, Smartphone oder Tablet verwendet. Durch die Auswertung an einem Computer ist es möglich, auf weitere Daten des Trägers zuzugreifen, welche üblicherweise in einem gesicherten Netzwerk abgelegt sind. Auch können die neu generierten Daten direkt dem Datensatz des jeweiligen Trägers hinzugefügt werden. Die Verwendung von Mobilgeräten wie Smartphones oder Tablets als Auswerteeinheit hat den Vorteil, dass die Daten und die Auswertung zeitnah verarbeitet werden können. Denn üblicherweise wird ein Mobil-Gerät fortwährend mitgeführt, so dass, beispielsweise ein akustisches Signal, das anzeigt, dass bei einem Träger, welcher das Sensormodul trägt, eine wichtige Änderung ergeben hat, direkt zu einer erforderlichen Maßnahme führen kann. Das kann die Trennung des ersten Teilmoduls vom zweiten Teilmoduls betreffen, es kann aber auch die Erkennung eines Sturzes, welcher durch die Auswertung der Beschleunigungsdaten erkannt wurde, betreffen.

In einem dritten Aspekt der Erfindung wird die Verwendung eines zweiten Teilmoduls zur Verbindung mit einem ersten Teilmodul in einem Sensormodul vorgeschlagen, wobei das erste Teilmodul mittels einer elektronischen Einheit Bewegungsdaten erfassen und übermitteln kann, und eine erste Struktur umfasst, und wobei das zweite Teilmodul ein Befestigungsmittel zur Befestigung an einem Träger umfasst, und eine zweite Struktur umfasst, welche mit der ersten Struktur beim Zusammendrücken eine reversible mechanische Verbindung ausbildet, so dass das erste Teilmodul und das zweite Teilmodul reversibel miteinander verbunden sind.

Bevorzugt wird ein Nahfeldkommunikations-Sender verwendet, welcher geeignet ist, Daten an einen Nahfeldkommunikations-Empfänger zu senden, wobei die Daten zur Verifizierung
a) der Funktionalität der Paarung des ersten Teilmoduls mit dem zweiten Teilmodul, und/oder
b) der korrekten Anbringung des ersten Teilmoduls und/oder
c) der Verwendung eines proprietären zweiten Teilmoduls
herangezogen werden.

### KURZE BESCHREIBUNG DER ZEICHNUNGEN

Weitere Merkmale und Vorteile der Erfindung ergeben sich aus der nachfolgenden Beschreibung bevorzugter Ausführungsbeispiele der Erfindung anhand der Zeichnungen. Darin zeigen schematisch:
- Figur 1A: eine erste Vorderseite eines ersten Teilmoduls des erfindungsgemäßen Sensormoduls.
- Figur 1B: eine erste Rückseite des ersten Teilmoduls des erfindungsgemäßen Sensormoduls.
- Figur 2A: eine zweite Vorderseite eines zweiten Teilmoduls des erfindungsgemäßen Sensormoduls.
- Figur 2B: eine zweite Rückseite des zweiten Teilmoduls des erfindungsgemäßen Sensormoduls.
- Figur 3: das erfindungsgemäße Sensormodul, aufgeklebt auf dem Rücken eines Trägers, und eine Auswerteeinheit.

### BESCHREIBUNG BEVORZUGTER AUSFÜHRUNGSBEISPIELE

Bei der nachfolgenden Beschreibung bevorzugter Ausführungsformen der vorliegenden Erfindung bezeichnen gleiche Bezugszeichen gleiche oder vergleichbare Komponenten.

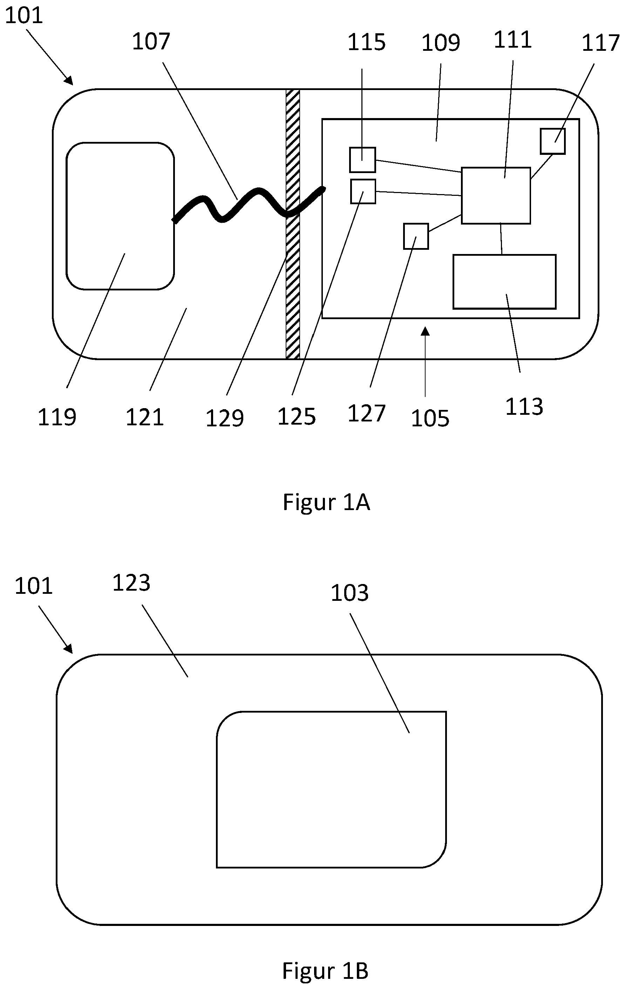

**Figur 1A** zeigt eine erste Vorderseite 121 eines ersten Teilmoduls 101 des erfindungsgemäßen Sensormoduls 10. Das erste Teilmodul 101 ist flächig ausgebildet und weist eine rechteckige Grundfläche mit abgerundeten Ecken, welche auch nicht-abgerundet sein können, auf. Ebenso kann die Grundfläche quadratisch oder eine andere Form aufweisen. Auf der ersten Vorderseite 121 ist eine elektronische Einheit 105 angeordnet, welche eine Leiterplatte 109 mit darauf angeordneten und elektrisch untereinander verbundenen elektronischen Komponenten 111, 113, 115, 117, 125, 127 umfasst. Die Leiterplatte 109 ist über eine flexible elektrische Leitung 107 mit einer Leistungsquelle 119 verbunden, beispielsweise einer Batterie, welche die Leiterplatte 109 und den darauf angeordneten elektronischen Komponenten 111, 113, 115, 117, 125, 127 mit Energie versorgt. Die Leiterplatte 109 kann auch als gedruckte Leiterplatte ausgeführt sein. Die auf der Leiterplatte 109 angeordneten elektronischen Komponenten umfassen einen oder mehrere Beschleunigungssensoren 115, einen Mikrocontroller 111, ein Kommunikationsmodul 113, einen Nahfeldkommunikations-Empfänger 117, einen kapazitiven Sensor 127, sowie ein GPS-Modul 125, wobei GPS globales Positionssystem bedeutet. Das Kommunikationsmodul 113 kann als GSM-Kommunikationsmodul ausgebildet sein, wobei GSM globaler Mobilfunkstandard bedeutet. Der kapazitive Sensor 127 weist zudem eine Kondensator-Elektrode auf. Im montierten Zustand des Sensormoduls 10, also wenn das erste Teilmodul 101 am zweiten Teilmodul 201 befestigt ist und das zweite Teilmodul 201 auf der Hautoberfläche des Trägers befestigt ist, kann zwischen der Kondensator-Elektrode und der Hautoberfläche des Trägers entsprechend eine Kapazität, da die Haut des Trägers als Elektrode wirkt, gemessen werden. Die in diesem montierten Zustand gemessene Kapazität kann hinsichtlich weiterer Messungen als Referenzwert herangezogen werden. Die elektronische Einheit 105 umfasst eine Vergleichseinheit (nicht dargestellt), welche ausgebildet ist, diesen Kapazitäts-Referenzwert mit einem aktuellen Kapazitätswert zu vergleichen. Wenn der aktuelle Kapazitätswert größer oder gleich dem Referenzwert ist löst die Vergleichseinheit entsprechend aus, dass die Daten der Beschleunigung, der Ortskoordinaten sowie der Verifizierung gemessen, erfasst und/oder übermittelt werden. Sollte der aktuelle Kapazitätswert kleiner als der Referenzwert sein, also das erste Teilmodul 101 vom zweiten Teilmodul 201 entfernt worden sein, oder auch das gesamte Sensormodul 10 von der Hautoberfläche des Trägers entfernt worden sein, so werden keine Daten der Beschleunigung, der Ortskoordinaten sowie der Verifizierung gemessen, erfasst und/oder übermittelt. Die elektronische Einheit 105 kann in dieser Situation in einen Ruhezustand übergehen, in welchem nur noch in einem gewünschten Intervall die Kapazitätswerte gemessen und mit dem Referenzwert verglichen werden. Sobald der aktuell gemessene Kapazitätswert wieder größer oder gleich dem Referenzwert ist, werden die Daten der Beschleunigung, der Ortskoordinaten sowie der Verifizierung gemessen, erfasst und/oder übermittelt.

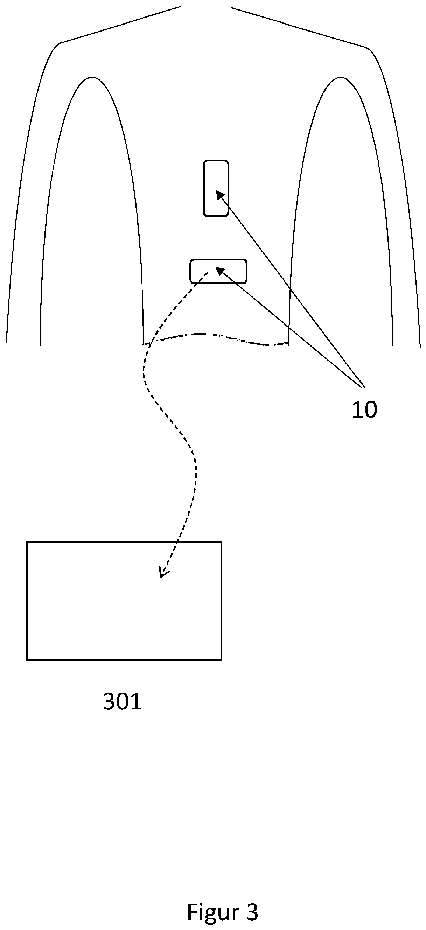

Mit dem einen oder den mehreren Beschleunigungssensoren 115 werden Beschleunigungsdaten gemessen und entsprechend an den Mikrocontroller 111 weitergeleitet. Durch die Verwendung mehrerer Beschleunigungssensoren 115 können Beschleunigungen in verschiedene Richtungen gemessen werden. Der Mikrocontroller 111 leitet die von dem wenigstens einen Beschleunigungssensor 115, dem GPS-Modul und dem Nahfeldkommunikations-Empfänger 117 erhaltenen Daten an das Kommunikationsmodul 113 weiter, welches die Daten an eine externe Auswerteeinheit 301 (siehe Figur 3) übermittelt. Ebenso werden die durch den kapazitiven Sensor gemessenen Kapazitäten über das Kommunikationsmodul 113 an die Auswerteeinheit 301 weitergeleitet.

Das erste Teilmodul 101 ist von einer Silikon-Ummantelung eingefasst. Die Silikon-Ummantelung sowie die Silikon-Struktur auf der ersten Vorderseite 121 kann mittels Spritzgusstechnik erzeugt werden. Dabei werden die Leistungsquelle 119 und die elektronische Einheit 105 von der Silikon-Ummantelung umschlossen. Bei einer rechteckigen Grundfläche mit einer Längs- und einer Querachse sind die Leistungsquelle 119 und die elektronische Einheit 105 entlang der Längsachse des Rechtecks beabstandet nebeneinander eingebettet. Zwischen der elektronischen Einheit 105 und der Leistungsquelle 119 ist quer zur Längsachse des Rechtecks ein mechanisch flexibler, bevorzugt linearer, Bereich 129 ausgebildet, beispielsweise in Form einer Vertiefung im Material. Dadurch ist das erste Teilmodul 101 entlang der Querachse des Rechtsecks, welche zwischen der angeordneten Leistungsquelle 109 und der Leiterplatte 119 verläuft, mechanisch flexibel, und kann sich während des Tragens durch den Träger an dessen Bewegungen anpassen. Der mechanisch flexible Bereich 129 ist im vorliegenden Ausführungsbeispiel auf der ersten Vorderseite 121 ausgebildet. Genauso kann der mechanisch flexible Bereich 129 auf der ersten Rückseite 123 oder sowohl auf der ersten Vorderseite 121 als auch auf der ersten Rückseite 123 gegenüberliegend ausgebildet sein.

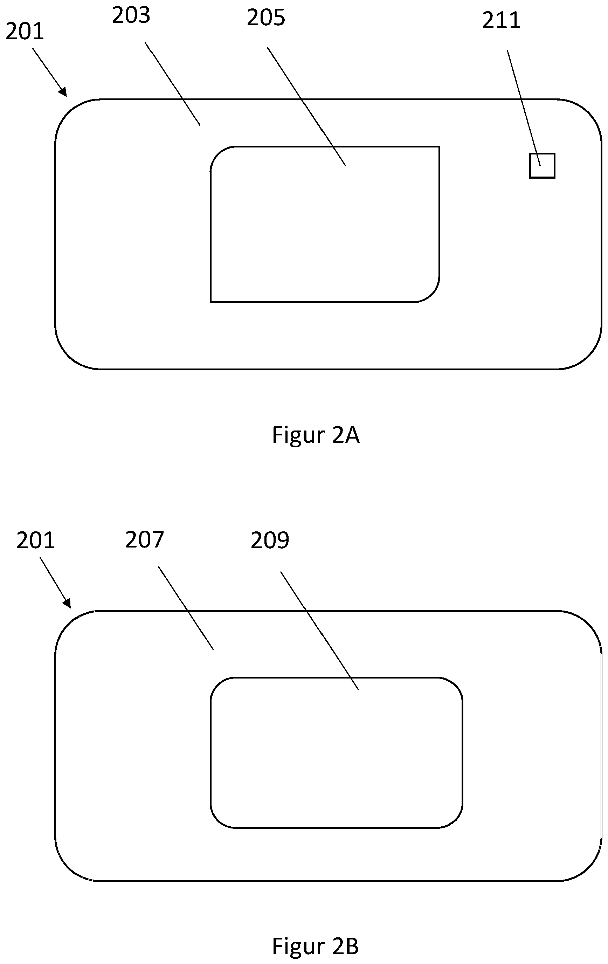

Der Nahfeldkommunikations-Empfänger 117 ist geeignet, die gesendeten Daten eines Nahfeldkommunikations-Senders 211, welcher am zweiten Teilmodul 201 (siehe Fig. 2A) angeordnet ist, zu empfangen. Der Nahfeldkommunikations-Empfänger 117 und der Nahfeldkommunikations-Sender 211 kommunizieren auf Basis des Nahfeldkommunikations-Standards (NFC-Standard) RFID, das Radiofrequenz-Identifikation bedeutet. Der Datenaustausch in der Nahfeldkommunikation erfolgt über elektromagnetische Induktion und ist daher nur bei geringen Reichweiten von wenigen Zentimetern bis etwa 10 cm möglich.

**Figur 1B** zeigt eine erste Rückseite 123 des ersten Teilmoduls 101, welche der ersten Vorderseite 121 abgewandt ist. Die erste Rückseite 123 umfasst eine erste Struktur 103, welche als Haken- oder Schlaufen-Struktur ausgebildet sein kann. Die erste Struktur 103 kann sich über einen Teilbereich der ersten Rückseite 123 oder auch über die gesamte erste Rückseite 123 erstrecken.

**Figur 2A** zeigt eine zweite Vorderseite 203 eines zweiten Teilmoduls 201. Auf der zweiten Vorderseite 203 ist eine zweite Struktur 205 ausgebildet, welche als Haken- oder Schlaufen-Struktur ausgebildet ist und das Gegenstück zur Haken- oder Schlaufenstruktur der ersten Struktur 103 des ersten Teilmoduls 101 bildet. Beim Zusammendrücken der ersten Struktur 103 mit der zweiten Struktur 203 verbinden sich die Haken- und Schlaufenstruktur entsprechend und eine mechanisch reversible Formschlussverbindung wird ausgebildet, beispielsweise ein Klett-Verschluss. Dadurch werden das erste Teilmodul 101 und das zweite Teilmodul 201 durch einen mechanischen reversiblen Formschluss miteinander verbunden. Die zweite Struktur 203 kann sich über einen Teilbereich der zweiten Vorderseite 203 oder auch über die gesamte zweite Vorderseite 203 erstrecken.

Die mechanisch reversible Formschlussverbindung ermöglicht die Abnahme des ersten Teilmoduls 101 für mögliche Modifikationen oder Reparaturen, während das zweite Teilmodul 201 durch die Klebeverbindung 209 an der jeweiligen Position am Körper eines Trägers haften bleibt. Auch ermöglicht dieser modulare Aufbau das erste Teilmodul 101 durch ein anderes Teilmodul ersetzen zu können.

Ferner ist auf der zweiten Vorderseite 203 ein Nahfeldkommunikations-Sender 211 angeordnet. Der Nahfeldkommunikations-Sender 211 kann auch auf der zweiten Rückseite 207 angeordnet sein, oder in das zweite Teilmodul 201 integriert sein, so dass der Nahfeldkommunikations-Sender 211 nach außen hin nicht sichtbar ist. Für eine erfolgreiche Datenübertragung ist es erforderlich, dass der Nahfeldkommunikations-Sender 211 in örtlicher Nähe, also in einem Abstand von weniger als 10 cm, zum Nahfeldkommunikations-Empfänger 117 angeordnet ist. Die Daten, welche der Nahfeldkommunikations-Sender 211 an den Nahfeldkommunikations-Empfänger 117 sendet, können hinsichtlich der Funktionalität der Paarung des ersten Teilmoduls 101 mit dem zweiten Teilmodul 201, der korrekten Anbringung des ersten Teilmoduls 101 am zweiten Teilmodul 201 bezüglich Vorder- und Rückseite und der Ausrichtung, sowie der Verwendung eines proprietäreren zweiten Teilmoduls 201 verifiziert werden. Ob eine funktionierende Paarung vorliegt, kann über die Funktionalität einer Nahfeldkommunikation an sich verifiziert werden. Wenn das erste Teilmodul 101 zu weit von dem zweiten Teilmodul 201 entfernt ist, also beispielsweise mehr als 10-15 cm, und keine Nahfeldkommunikation mehr möglich ist kann auch keine Paarung erfolgen. Das Gleiche gilt, wenn das erste Teilmodul 101 nicht korrekt mit dem zweiten Teilmodul 201 verbunden ist, also wenn die Grundflächen der beiden Teilmodule 101, 201 um 180° zueinander verdreht wurden. In diesem Fall wären zwar das erste Teilmodul 101 und das zweite Teilmodul 201 über die mechanisch reversible Verbindung verbunden, aber der Nahfeldkommunikations-Sender 211 wäre nicht in unmittelbarer Nähe zum Nahfeldkommunikations-Empfänger 117 angeordnet, so dass keine Nahfeldkommunikation erfolgen kann. Ferner können die durch den Nahfeldkommunikations-Sender 211 gesendeten Daten ein Erkennungssignal enthalten, um sicherzustellen, dass es sich beim zweiten Teilmodul 201 um ein Original-Teilmodul und um keinen Nachbau handelt.

Zusätzlich oder alternativ ist es auch möglich, die Ermittlung des Kapazitäts-Referenzwertes an eine erfolgreiche Paarung zu koppeln. Sobald durch den Nahfeldkommunikations-Sender 211 und den Nahfeldkommunikations-Empfänger 117 eine Paarung erfolgt ist, wird eine erste Kapazitätsmessung des kapazitiven Sensors ausgelöst, welche als Kapazitäts-Referenzwert verwendet werden kann.

**Figur 2B** zeigt eine zweite Rückseite 207 des zweiten Teilmoduls 201, welche der zweiten Vorderseite 203 abgewandt ist. Die zweite Rückseite 207 umfasst eine klebende Schicht 209, welche an der menschlichen Haut haften bleibt, und über welche das zweite Teilmodul 201 am Körper des Trägers befestigt wird. Die klebende Schicht 209 kann sich über einen Bereich der zweiten Rückseite 207 oder aber auch über die gesamte zweite Rückseite 207 erstrecken. Die klebende Schicht kann als biokompatibler dehnbarer Pflaster-Klebestreifen ausgebildet sein.

**Figur 3** zeigt das erfindungsgemäße Sensormodul 10, beispielhaft an zwei verschiedenen Positionen auf dem Rücken einer Person angebracht. Es ist aber auch möglich, das Sensormodul 10 an anderen Stellen des Rückens oder auch an der Brust oder im Bauchbereich anzubringen. Bevorzugt wird das Sensormodul 10, ausgehend von einer der Längsachse einer rechtwinkligen Grundfläche, entlang der Wirbelsäule oder im rechten Winkel hierzu angeordnet. Dies hat den Vorteil, dass flexible Vorrichtungskomponenten, also die flexible Leiterverbindung 119 und der flexible lineare Bereich 129 der Silikon-Ummantelung, den Bewegungen des Trägers wenigstens teilweise folgen können.

Ferner ist eine separat angeordnete Auswerteeinheit 301 gezeigt, welche Daten des Kommunikationsmoduls 113 der elektronischen Einheit 105 empfangen und auswerten kann. Die über das Kommunikationsmodul 113 an die Auswerteeinheit 301 übermittelten Daten, basierend auf Daten der Beschleunigungssensoren 115, des Nahfeldkommunikations-Senders 211 sowie des kapazitiven Sensors, werden hinsichtlich Trageerkennung, Sturzerkennung, Lageerkennung, sowie Dekubituserkennung ausgewertet. Ferner ist eine Ortung und eine Aktivitätsanalyse über Daten des GPS-Moduls 125 möglich, welche ebenfalls über das Kommunikationsmodul 113 an die Auswerteeinheit 301 gesendet werden. Die Auswertung der Daten erfolgt an einem Computer und/oder einem Mobilgerät wie beispielsweise ein Tablet oder ein Smartphone. Dadurch erfolgt eine Auswertung in einer angebundenen Infrastruktur und gegebenenfalls erforderliche Maßnahmen können zeitnah erfolgen. Zudem können durch einen Computer, der mit einem gegen den Zugriff Dritter gesicherten Netzwerk verbunden ist, die Daten für den jeweiligen Träger zentral hinterlegt werden. Die Auswertung der Daten an einem Mobilgerät hat den Vorteil, dass die Kenntnisnahme eines Vorfalls, wie beispielsweise eines Sturzes, unmittelbar erkannt wird, da ein Mobilgerät üblicherweise von dem Nutzer, also einer autorisierten Fachkraft, mitgeführt wird.

Das erfindungsgemäße Sensormodul 10 sowie das Pflegeset kommen bevorzugt in Einrichtungen für pflegebedürftige Menschen zum Einsatz, wie Pflegeheimen oder Altersheimen, in welchen die pflegebedürftigen Menschen ein jeweiliges Sensormodul 10 tragen und Pflege-Fachkräfte die erfassten Daten auswerten.

### BEZUGSZEICHENLISTE

- 10: Sensormodul
- 101: Erstes Teilmodul
- 103: Erste Struktur
- 105: Elektronische Einheit
- 107: Flexible Leiterverbindung
- 109: Leiterplatte
- 111: Mikrocontroller
- 113: Kommunikationsmodul
- 115: Beschleunigungssensor
- 117: Nahfeldkommunikations-Empfänger
- 119: Leistungsquelle
- 121: Erste Vorderseite
- 123: Erste Rückseite
- 125: GPS Modul
- 127: Kapazitiver Sensor
- 129: Flexibler Bereich
- 201: Zweites Teilmodul
- 203: Zweite Vorderseite
- 205: Zweite Struktur
- 207: Zweite Rückseite
- 209: Klebende Schicht
- 211: Nahfeldkommunikations-Sender
- 301: Auswerteeinheit

## Patentansprüche

1. Sensormodul (10), umfassend
- ein erstes Teilmodul (101), umfassend
a) eine elektronische Einheit (105) zur Erfassung und Übermittlung von Bewegungsdaten, und
b) eine erste Struktur (103), und
- ein zweites Teilmodul (201), umfassend
a) ein Befestigungsmittel zur Befestigung des zweiten Teilmoduls (201) an einem Träger, und
b) eine zweite Struktur (205),
wobei die erste Struktur (103) mit der zweiten Struktur (205) eine reversible mechanische Verbindung ausbilden kann, so dass das erste Teilmodul (101) und das zweite Teilmodul (201) reversibel miteinander verbunden werden können,
wobei die erste Struktur (103) eines aus der Gruppe Haken-Struktur oder Schlaufen-Struktur umfasst und die zweite Struktur (205) das andere aus der Gruppe Haken-Struktur oder Schlaufen-Struktur umfasst, welche beim Zusammendrücken der ersten Struktur (103) mit der zweiten Struktur (205) die reversible mechanische Verbindung ausbilden,
wobei die reversible mechanische Verbindung einen Klettverschluss umfasst,
wobei das Befestigungsmittel eine klebende Schicht (209) enthält, und
wobei die klebende Schicht (209) an der menschlichen Haut haftbar ist.

2. Sensormodul (10) nach Anspruch 1, wobei das zweite Teilmodul (201) als dehnbarer Klebestreifen ausgebildet ist.

3. Sensormodul (10) nach einem der Ansprüche 1 oder 2, wobei die elektronische Einheit (105) wenigstens einen Beschleunigungssensor (115) zur Messung von Beschleunigungsdaten und/oder ein GPS-Modul (125) zur Erfassung und Übermittlung von Ortskoordinaten des ersten Teilmoduls (101) bzw. des Sensormoduls (10) umfasst.

4. Sensormodul (10) nach einem der Ansprüche 1 bis 3, wobei
- das zweite Teilmodul (201) einen Nahfeldkommunikations-Sender (211) zum Senden von Daten, und
- das erste Teilmodul (101) einen Nahfeldkommunikations-Empfänger (117) zum Empfangen der Daten umfasst, wobei
- die Daten zur Verifizierung
a) der Funktionalität der Paarung des ersten Teilmoduls (101) mit dem zweiten Teilmodul (201), und/oder
b) der korrekten Anbringung des ersten Teilmoduls (101) und/oder
c) der Verwendung eines proprietären zweiten Teilmoduls (201)
herangezogen werden.

5. Sensormodul (10) nach einem der Ansprüche 1 bis 4, wobei das erste Teilmodul (101) einen kapazitiven Sensor (127) zur Messung von Kapazitätswerten zwischen einer dem kapazitiven Sensor (127) zugehörigen Kondensator-Elektrode und der Hautoberfläche des Trägers umfasst.

6. Sensormodul (10) nach einem der Ansprüche 1 bis 5, wobei die elektronische Einheit (105) eine Vergleichseinheit umfasst, welche ausgebildet ist, einen Kapazitäts-Referenzwert mit einem aktuellen Kapazitätswert zu vergleichen, und auszulösen, dass Daten nur dann gemessen, erfasst und/oder übermittelt werden, wenn der aktuelle Kapazitätswert entweder a) größer oder gleich oder b) kleiner oder gleich dem Referenzwert ist.

7. Sensormodul (10) nach einem der Ansprüche 1 bis 5, wobei die elektronische Einheit (105) eine Vergleichseinheit umfasst, welche ausgebildet ist, einen Kapazitäts-Referenzwert mit einem aktuellen Kapazitätswert zu vergleichen, und auszulösen, dass die Daten der Beschleunigung, der Ortskoordinaten sowie der Verifizierung nur dann gemessen, erfasst und/oder übermittelt werden, wenn der aktuelle Kapazitätswert kleiner oder gleich dem Referenzwert ist, oder
die elektronische Einheit (105) eine Vergleichseinheit umfasst, welche ausgebildet ist, einen Kapazitäts-Referenzwert mit einem aktuellen Kapazitätswert zu vergleichen, und auszulösen, dass die Daten der Beschleunigung, der Ortskoordinaten sowie der Verifizierung nur dann gemessen, erfasst und/oder übermittelt werden, wenn der aktuelle Kapazitätswert größer oder gleich dem Referenzwert ist.

8. Sensormodul (10) nach einem der Ansprüche 1 bis 7, wobei die elektronische Einheit (105) ein Kommunikationsmodul (113) umfasst, mit welchem die erfassten Daten an eine Auswerteeinheit außerhalb des Sensormoduls (10) übermittelt werden.

9. Sensormodul (10) nach einem der Ansprüche 1 bis 8, wobei
das erste Teilmodul (101) eine flexible Leiterverbindung (107) umfasst, welche die elektronische Einheit (105) mit einer Leistungsquelle (119) verbindet, welche die elektronische Einheit (105) mit Energie versorgt, und
das erste Teilmodul (101) eine Ummantelung aus einem flexiblen Material umfasst, welches vorzugsweise mittels eines Gießverfahrens die Leistungsquelle (119) und die elektronische Einheit (105) umschließt, so dass die Leistungsquelle (119) und die elektronische Einheit (105) mit der Ummantelung einen nichtlösbaren Formschluss ausbilden, wobei die Ummantelung wenigstens einen flexiblen, vorzugsweise linearen, Bereich (129) umfasst, welcher eine Biegeachse ausbildet, um welche sich das erste Teilmodul (101) biegen lässt, wobei der flexible Bereich quer zur flexiblen Leiterverbindung (107) verläuft, so dass bevorzugt bei einer Biegung des ersten Teilmoduls (101) um den linearen Bereich die flexible Leiterverbindung (107) ebenfalls gebogen wird.

10. Sensormodul (10) nach Anspruch 9, wobei das Sensormodul (10) so angepasst ist, dass es ausgehend von einer Längsachse einer rechtwinkligen Grundfläche mit abgerundeten Ecken im rechten Winkel zur Wirbelsäule des Trägers so angeordnet werden kann, dass der flexible Bereich (129) den Bewegungen des Trägers wenigstens teilweise folgen kann.

11. Sensormodul (10) nach einem der Ansprüche 1 bis 10, wobei das Sensormodul (10) dazu ausgebildet ist, am Rücken, an der Brust oder im Bauchbereich des Trägers befestigt zu werden.

12. Pflegeset, umfassend
- ein Sensormodul (10) nach einem der Ansprüche 1 bis 11**,** und
a) eine von dem Sensormodul (10) getrennt angeordnete elektronische Auswerteeinheit (301) mit einer Software, mit welcher die vom Sensormodul (10) gemessenen Daten ausgewertet und angezeigt werden,
wobei die Auswerteeinheit (301) dazu ausgebildet ist, die gemessenen Daten zu empfangen, oder
b) ein Computerprogrammprodukt, das, wenn auf einer elektronischen Auswerteeinheit installiert und ausgeführt, eine Software bildet, mit welcher die vom Sensormodul (10) gemessenen Daten ausgewertet und angezeigt werden,
wobei die Auswerteeinheit (301) dazu ausgebildet ist, die gemessenen Daten zu empfangen.

13. Verwendung eines ersten Teilmoduls (101) und eines zweiten Teilmoduls (201) zur Verbindung mit dem ersten Teilmodul (101) in einem Sensormodul (10) mittels Klettverschluss, wobei das erste Teilmodul (101) mittels einer elektronischen Einheit (105) Bewegungsdaten erfassen und übermitteln kann, und eine erste Struktur (103) umfasst,
- wobei das zweite Teilmodul (201)
a) ein Befestigungsmittel zur Befestigung an einem Träger umfasst,
wobei das Befestigungsmittel eine klebende Schicht (209) enthält,
wobei die klebende Schicht (209) an der menschlichen Haut haftbar ist, und
b) eine zweite Struktur (205) umfasst, welche mit der ersten Struktur (103) beim Zusammendrücken eine reversible mechanische Verbindung ausbildet, so dass das erste Teilmodul (101) und das zweite Teilmodul (201) reversibel miteinander verbunden sind,
- wobei die erste Struktur (103) eines aus der Gruppe Haken-Struktur oder Schlaufen-Struktur umfasst und die zweite Struktur (205) das andere aus der Gruppe Haken-Struktur oder Schlaufen-Struktur umfasst, welche beim Zusammendrücken der ersten Struktur (103) mit der zweiten Struktur (205) die reversible mechanische Verbindung ausbilden, und
- wobei die reversible mechanische Verbindung einen Klettverschluss umfasst.

14. Verwendung nach Anspruch 13, wobei das zweite Teilmodul (201) einen Nahfeldkommunikations-Sender (211) umfasst, welcher geeignet ist, Daten an einen Nahfeldkommunikations-Empfänger (117) zu senden, wobei die Daten zur Verifizierung
a) der Funktionalität der Paarung des ersten Teilmoduls (101) mit dem zweiten Teilmodul (201), und/oder
b) der korrekten Anbringung des ersten Teilmoduls (101) und/oder
c) der Verwendung eines proprietären zweiten Teilmoduls (201) herangezogen werden.

15. Verwendung nach Anspruch 13 oder 14, wobei das zweite Teilmodul (201) zur Befestigung am Rücken, an der Brust oder im Bauchbereich eines Trägers ausgebildet ist.

## Claims

1. A sensor module (10) comprising
- a first submodule (101) comprising
a) an electronic unit (105) for capturing and transmitting movement data, and
b) a first structure (103), and
- a second submodule (201) comprising
a) a fastening means for fastening the second submodule (201) to a wearer, and
b) a second structure (205),
wherein the first structure (103) can form a reversible mechanical connection with the second structure (205), with the result that the first submodule (101) and the second submodule (201) can be reversibly connected to one another,
wherein the first structure (103) comprises one from the group of hook structure or loop structure and the second structure (205) comprises the other from the group of hook structure or loop structure, which structures form a reversible mechanical connection when the first structure (103) is pressed together with the second structure (205),
wherein the reversible mechanical connection comprises a hook-and-loop fastening,
wherein the fastening means comprises an adhesive layer (209), and
wherein the adhesive layer (209) can adhere to the human skin.

2. The sensor module (10) according to claim 1, wherein the second submodule (201) is in the form of a stretchy adhesive strip.

3. The sensor module (10) according to one of claims 1 or 2, wherein the electronic unit (105) comprises at least one acceleration sensor (115) for measuring acceleration data and/or a GPS module (125) for capturing and transmitting spatial coordinates of the first submodule (101) or of the sensor module (10).

4. The sensor module (10) according to one of claims 1 to 3, wherein
- the second submodule (201) comprises a near-field communication transmitter (211) for transmitting data, and
- the first submodule (101) comprises a near-field communication receiver (117) for receiving the data, wherein
- the data are used to verify
a) the functionality of the pairing of the first submodule (101) with the second submodule (201), and/or
b) the correct attachment of the first submodule (101), and/or
c) the use of a proprietary second submodule (201).

5. The sensor module (10) according to one of claims 1 to 4, wherein the first submodule (101) comprises a capacitive sensor (127) for measuring capacitance values between a capacitor electrode belonging to the capacitive sensor (127) and the wearer's skin surface.

6. The sensor module (10) according to one of claims 1 to 5, wherein the electronic unit (105) comprises a comparison unit which is designed to compare a capacitance reference value with a present capacitance value and to prompt data to be measured, captured and/or transmitted only when the present capacitance value is either a) greater than or equal to or b) less than or equal to the reference value.

7. The sensor module (10) according to one of claims 1 to 5, wherein the electronic unit (105) comprises a comparison unit which is designed to compare a capacitance reference value with a present capacitance value and to prompt the data relating to the acceleration, the spatial coordinates and the verification to be measured, captured and/or transmitted only when the present capacitance value is less than or equal to the reference value, or
the electronic unit (105) comprises a comparison unit which is designed to compare a capacitance reference value with a present capacitance value and to prompt the data relating to the acceleration, the spatial coordinates and the verification to be measured, captured and/or transmitted only when the present capacitance value is greater than or equal to the reference value.

8. The sensor module (10) according to one of claims 1 to 7, wherein the electronic unit (105) comprises a communication module (113) which is used to transmit the captured data to an evaluation unit outside the sensor module (10).

9. The sensor module (10) according to one of claims 1 to 8, wherein
the first submodule (101) comprises a flexible conductor connection (107) which connects the electronic unit (105) to a power source (119) which supplies the electronic unit (105) with energy, and
the first submodule (101) comprises a sheath made of a flexible material which preferably encloses the power source (119) and the electronic unit (105) by means of a casting process, with the result that the power source (119) and the electronic unit (105) form a non-releasable form fit with the sheath, wherein the sheath comprises at least one flexible, preferably linear, region (129) which forms a bending axis, about which the first submodule (101) can be bent, wherein the flexible region runs transversely with respect to the flexible conductor connection (107), with the result that the flexible conductor connection (107) is preferably likewise bent when bending the first submodule (101) around the linear region.

10. The sensor module (10) according to claim 9, wherein the sensor module (10) is adapted so that, based on a longitudinal axis of a rectangular base with rounded corners orthogonal to the wearer's spinal column, it can be positioned in such a way that the flexible region can at least partially follow the wearer's movements.

11. The sensor module (10) according to one of claims 1 to 10, wherein the sensor module (10) is designed to be attached to the wearer's back, chest, or abdominal region.

12. A care set comprising
- a sensor module (10) according to one of claims 1 to 11, and
a) an electronic evaluation unit (301) which is arranged separately from the sensor module (10) and has software which is used to evaluate and display the data measured by the sensor module (10),
wherein the evaluation unit (301) is designed to receive the measured data, or
b) a computer program product which, when installed and executed on an electronic evaluation unit, forms software which is used to evaluate and display the data measured by the sensor module (10),
wherein the evaluation unit (301) is designed to receive the measured data.

13. A use of a first submodule (101) and a second submodule (201) for connection to the first submodule (101) in a sensor module (10) by means of a hook-and-loop fastening, wherein the first submodule (101) can capture and transmit movement data by means of an electronic unit (105) and comprises a first structure (103),
- wherein the second submodule (201)
a) comprises a fastening means for fastening to a wearer,
wherein the fastening means comprises an adhesive layer (209),
wherein the adhesive layer (209) can adhere to the human skin, and
b) comprises a second structure (205) which forms a reversible mechanical connection with the first structure (103) when being pressed together, with the result that the first submodule (101) and the second submodule (201) are reversibly connected to one another,
- wherein the first structure (103) comprises one from the group of hook structure or loop structure and the second structure (205) comprises the other from the group of hook structure or loop structure, which structures form a reversible mechanical connection when the first structure (103) is pressed together with the second structure (205), and
- wherein the reversible mechanical connection comprises a hook-and-loop fastening.

14. The use according to claim 13, wherein the second submodule (201) comprises a near-field communication transmitter (211), which is suitable for transmitting data to a near-field communication receiver (117), wherein the data are used to verify
a) the functionality of the pairing of the first submodule (101) with the second submodule (201), and/or
b) the correct attachment of the first submodule (101), and/or
c) the use of a proprietary second submodule (201).

15. The use according to claim 13 or 14, wherein the second submodule (201) is designed to be attached to the wearer's back, chest, or abdominal region.

## Revendications

1. Module capteur (10) comprenant
- un premier sous-module (101) comprenant
a) une unité électronique (105) destinée à l'enregistrement et à la transmission de données de mouvement, et
b) une première structure (103), et
- un deuxième sous-module (201) comprenant
a) un moyen de fixation destiné à fixer le deuxième sous-module (201) sur un porteur, et
b) une deuxième structure (205),
la première structure (103) pouvant former une liaison mécanique réversible avec la deuxième structure (205), de sorte que le premier sous-module (101) et le deuxième sous-module (201) puissent être reliés entre eux de manière réversible,
la première structure (103) comprenant l'une des structures du groupe « structure à crochets » ou « structure à boucles » et la deuxième structure (205) comprenant l'autre des structures du groupe « structure à crochets » ou « structure à boucles », lesquelles, lors de la compression de la première structure (103) contre la deuxième structure (205) forment la liaison mécanique réversible,
la liaison mécanique réversible comprenant une fermeture autoagrippante,
le moyen de fixation comprenant une couche adhésive (209), et
la couche adhésive (209) pouvant adhérer à la peau humaine.

2. Module capteur (10) selon la revendication 1, dans lequel le deuxième sous-module (201) est réalisé sous la forme d'une bande adhésive extensible.

3. Module capteur (10) selon l'une des revendications 1 ou 2, dans lequel l'unité électronique (105) comprend au moins un capteur d'accélération (115) destiné à mesurer des données d'accélération et/ou un module GPS (125) destiné à enregistrer et à transmettre les coordonnées géographiques du premier sous-module (101) ou du module capteur (10).

4. Module capteur (10) selon l'une des revendications 1 à 3, dans lequel
- le deuxième sous-module (201) comprend un émetteur de communication en champ proche (211) destiné à émettre des données, et
- le premier sous-module (101) comprend un récepteur de communication en champ proche (117) destiné à recevoir les données, dans lequel
- les données sont utilisées pour vérifier
a) le bon fonctionnement de l'appairage du premier sous-module (101) avec le deuxième sous-module (201), et/ou
b) la mise en place correcte du premier sous-module (101), et/ou
c) l'utilisation d'un deuxième sous-module propriétaire (201).

5. Module capteur (10) selon l'une des revendications 1 à 4, dans lequel le premier sous-module (101) comprend un capteur capacitif (127) destiné à mesurer des valeurs de capacité entre une électrode de condensateur associée au capteur capacitif (127) et la surface cutanée du porteur.

6. Module capteur (10) selon l'une des revendications 1 à 5, dans lequel l'unité électronique (105) comprend une unité de comparaison qui est conçue pour comparer une valeur de référence de capacité à une valeur de capacité actuelle, et déclencher la mesure, l'enregistrement et/ou la transmission de données uniquement lorsque la valeur de capacité actuelle est soit a) supérieure ou égale, soit b) inférieure ou égale à la valeur de référence.

7. Module capteur (10) selon l'une des revendications 1 à 5, dans lequel l'unité électronique (105) comprend une unité de comparaison qui est conçue pour comparer une valeur de référence de capacité à une valeur de capacité actuelle, et déclencher la mesure, l'enregistrement et/ou la transmission des données d'accélération, des coordonnées de position ainsi que de la vérification uniquement lorsque la valeur de capacité actuelle est inférieure ou égale à la valeur de référence, ou
l'unité électronique (105) comprend une unité de comparaison qui est conçue pour comparer une valeur de référence de capacité à une valeur de capacité actuelle, et déclencher la mesure, l'enregistrement et/ou la transmission des données d'accélération, des coordonnées de position ainsi que de la vérification uniquement lorsque la valeur de capacité actuelle est supérieure ou égale à la valeur de référence.

8. Module capteur (10) selon l'une des revendications 1 à 7, dans lequel l'unité électronique (105) comprend un module de communication (113) à l'aide duquel les données enregistrées sont transmises à une unité d'évaluation située à l'extérieur du module capteur (10).

9. Module capteur (10) selon l'une des revendications 1 à 8, dans lequel
le premier sous-module (101) comprend une connexion conductrice flexible (107) qui relie l'unité électronique (105) à une source d'alimentation (119) qui alimente l'unité électronique (105) en énergie, et
le premier sous-module (101) comprend une gaine en matériau souple qui, de préférence au moyen d'un procédé de moulage, entoure la source d'alimentation (119) et l'unité électronique (105), de sorte que la source d'alimentation (119) et l'unité électronique (105) forment avec la gaine un assemblage par complémentarité de forme non amovible, la gaine comprenant au moins une zone flexible, de préférence linéaire, (129) qui forme un axe de flexion autour duquel le premier sous-module (101) peut être courbé, la zone flexible s'étendant transversalement par rapport à la connexion conductrice flexible (107), de sorte que, de préférence, lors d'une flexion du premier sous-module (101) autour de la zone linéaire, la connexion conductrice flexible (107) est également courbée.

10. Module capteur (10) selon la revendication 9, dans lequel le module capteur (10) est adapté de manière à pouvoir être disposé, à partir d'un axe longitudinal d'une base rectangulaire aux angles arrondis et perpendiculaire à la colonne vertébrale du porteur, de telle sorte que la zone flexible (129) puisse suivre au moins partiellement les mouvements du porteur.

11. Module capteur (10) selon l'une des revendications 1 à 10, le module capteur (10) étant conçu pour être fixé sur le dos, la poitrine ou la région abdominale du porteur.

12. Kit de soins comprenant
- un module capteur (10) selon l'une des revendications 1 à 11, et
a) une unité d'évaluation électronique (301) disposée séparément du module capteur (10) pourvue d'un logiciel permettant d'évaluer et d'afficher les données mesurées par le module capteur (10),
l'unité d'évaluation (301) étant conçue pour recevoir les données mesurées, ou
b) un produit logiciel qui, lorsqu'il est installé et exécuté sur une unité d'évaluation électronique, constitue un logiciel permettant d'évaluer et d'afficher les données mesurées par le module capteur (10),
l'unité d'évaluation (301) étant conçue pour recevoir les données mesurées.

13. Utilisation d'un premier sous-module (101) et d'un deuxième sous-module (201) destiné à être relié au premier sous-module (101) dans un module capteur (10) au moyen d'une fermeture autoagrippante, le premier sous-module (101) pouvant enregistrer et transmettre des données de mouvement au moyen d'une unité électronique (105), et comprenant une première structure (103),
- le deuxième sous-module (201)
a) comprenant un moyen de fixation destiné à être fixé sur un porteur,
le moyen de fixation comportant une couche adhésive (209),
la couche adhésive (209) pouvant adhérer à la peau humaine, et
b) comprenant une deuxième structure (205) qui, lorsqu'elle est pressée contre la première structure (103), forme une liaison mécanique réversible, de sorte que le premier sous-module (101) et le deuxième sous-module (201) sont reliés entre eux de manière réversible,
- la première structure (103) comprenant l'une des structures du groupe « structure à crochets » ou « structure à boucles », et la deuxième structure (205) comprenant l'autre des structures du groupe « structure à crochets » ou « structure à boucles », lesquelles forment la liaison mécanique réversible lors de la compression de la première structure (103) contre la deuxième structure (205), et
- la liaison mécanique réversible comprenant une fermeture autoagrippante.

14. Utilisation selon la revendication 13, dans laquelle le deuxième sous-module (201) comprend un émetteur de communication en champ proche (211) apte à transmettre des données à un récepteur de communication en champ proche (117), les données devant être utilisées pour vérifier
a) le bon fonctionnement de l'appairage du premier sous-module (101) avec le deuxième sous-module (201), et/ou
b) la mise en place correcte du premier sous-module (101), et/ou
c) l'utilisation d'un deuxième sous-module propriétaire (201).

15. Utilisation selon la revendication 13 ou 14, dans laquelle le deuxième sous-module (201) est conçu pour être fixé sur le dos, la poitrine ou la région abdominale d'un porteur.
